# EUROPÄISCHE PATENTSCHRIFT

(11) **EP 0 715 837 B1**
(45) Veröffentlichungstag und Bekanntmachung des Hinweises auf die Patenterteilung: **08.12.1999**
(21) Anmeldenummer: 95102932.1
(22) Anmeldetag: 02.03.1995
(51) Int. Cl.: A61F 7/02

(54) **Therapeutische Kalt-Warmkompresse**
Therapeutic hot and cold compress
Compresse thérapeutique chaude-froide

(30) Priorität: 11.11.1994 DE 4440327
(43) Veröffentlichungstag der Anmeldung: 12.06.1996
(73) Patentinhaber: Beinio, Heinz, D-47551 Huisberden (DE)
(72) Erfinder: Beinio, Heinz, D-47551 Huisberden (DE)
(74) Vertreter: Herrmann-Trentepohl, Werner, Dipl.-Ing.

(56) Entgegenhaltungen:
- DE-A- 3 411 357
- DE-U- 8 622 573
- FR-A- 2 543 670
- US-A- 4 958 635

## Beschreibung

Die Erfindung bezieht sich auf eine therapeutische Kalt-Warmkompresse gemäß dem Oberbegriff des Anspruches 1.

Die erfindungsgemäße Kalt-Wärmekompresse läßt sich infolge ihrer sowohl mit Kälte als auch mit Wärme aufladbaren Trägersubstanz vielseitig anwenden, darunter zum Therapieren von Verstauchungen, Zerrungen, Verrenkungen, Schwellungen, Verbrennungen, Blutergüssen, Krämpfen, Hexenschuß, Kopfschmerzen, Migräne und Arthritis sowie zur Bekämpfung von Fieber benutzen. Ihre Anwendung ist verhältnismäßig einfach, weil sie infolge des Beutels und des diesen im wesentlichen ausfüllenden flüssigen Substrates flexibel ist und sich dadurch beim Auflegen auf eine zu therapierende Körperpartie deren Form anpassen kann. Diese Kalt-Warmkompresse läßt sich in einem normalen Haushaltskühlschrank mit Kälte und in heißem Wasser mit Wärme aufladen, bevor sie zweckmäßig nach einbringen in eine aus einem dünnen Tuch oder ähnlichem bestehenden Kompressenhülle auf die Haut der zu therapierenden Körperpartie aufgebracht wird.

Die Trägersubstanz übernimmt in der erfindungsgemäßen therapeutischen Kalt-Warmkompresse die Aufgabe, die aufgeladene Wärme bzw. Kälte über einen längeren Zeitraum zu speichern und infolge des Wärmegefälles gegenüber der Haut Wärme bzw. Kälte an die zu therapierende Körperpartie abzugeben. Das Kältemittel dient dazu, das flüssige Substrat beim Aufladen der Trägersubstanz mit Kälte nicht einfrieren zu lassen, so daß der Beutel dicht und flexibel bleibt.

Bekannt sind therapeutische Kalt-Warmkompressen, welche als Wärme- bzw. Kälteträger ein Gel benutzen, das aus Carboxyethylcellulose besteht und mit Glykol vermischt wird, das als Kältemittel dient. Der Beutel bildet ein Kissen von einer meistens transparenten Kunststoffolie.

Solche therapeutischen Kalt-Warmkompressen befriedigen jedoch häufig in der Praxis nicht. Einerseits sind die bislang verwendbaren Gele nur begrenzt mit Wärme oder Kälte aufladbar. Andererseits gibt die Trägersubstanz nur ein verhältnismäßig geringen Anteil ihrer aufgeladenen Wärme bzw. Kälte an die zu therapierende Körperpartie und einen häufig größerem Anteil an die Umgebung ab. Insgesamt ist daher der gewünschte physikalische Effekt der Aufheizung bzw. Kühlung der zu therapierenden Körperpartie unbefriedigend.

Auserdem ist aus DE-U-8622573 eine Kalt-Warmkompresse bekannt gemäß dem Oberbegriff des Anspruchs 1.

Die Informationsschrift 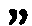 Natur tut gut K-MED Moortherapie" der Kampmann MED GmbH in W-4450 Lingen 1, Deutschland, veröffentlicht im April 1992, offenbart eine Kältepackung, bestehend aus im Urzustand belassenem, homogenisiertem Naturmoor mit einem Feuchtigkeitsgehalt von 88 - 92 %, ohne jeglichen Zusatz. Auch werden Kälteträger auf Naturmoorbasis für die kryotherapie, im Minusbereich von bis zu -20° C, offenbart. Die Kälteträger bestehen dabei aus abgeschlossenen Kunststoffbeuteln, in die das Naturmoor eingebracht ist.

Der Erfindung liegt das Problem zugrunde, die Wirksamkeit einer therapeutischen Kalt-Warmkompresse durch eine Steigerung des Anteils der ihrer Trägersubstanz aufgeladenen Wärme oder Kälte, der auf die zu therapierende Körperpartie übertragen wird zu verbessern.

Gemäß einer im Anspruch 1 wiedergegebenen Aufgabenlösung der Erfindung wird die Trägersubstanz in ihrer Zusammensetzung und Anordnung im Beutel der Kompresse gegenüber dem Stand der Technik geändert und dadurch verbessert. Der erfindungsgemäße in der Trägersubstanz das Substrat bildende Humintorf hat ein erheblich gegenüber den bisher verwendeten flüssigen Substraten gesteigertes Wärme- und Kältespeichervermögen. Zwar gehört die Verwendung von Torf verschiedener Provinienz zu therapeutischen Zwecken in warmen Kompressen bereits zum Stand der Technik. Hierbei werden jedoch die dampf- bis gasförmigen Bestandteile des erhitzten Torfes zusammen mit der gespeicherten Wärme für die Therapie eingesetzt. In Kaltkompressen ist Torf bislang als flüssiges Substrat unbekannt. Erfindungsgemäß wird in der neuen therapeutischen Kalt-Warmkompresse der in der Trägersubstanz als Substrat verwendete Humintorf dampfdicht abgeschlossen untergebracht. Dadurch ist gewährleistet, daß lediglich die Wärme für den therapeutischen Zweck genutzt wird, während alle anderen Bestandteile des Humintorfs in der Trägersubstanz unabhängig von der Anzahl der Therapievorgänge verbleiben. Da erfindungsgemäß ferner der Humintorf luftblasenfrei in der Kompresse untergebracht wird, entwickelt die Kompresse über ihre volle Größe die gewünschte Wärme- bzw. Kälteabgabe und ermöglicht eine erhebliche Aufheizung, ohne daß der Beutel infolge Luftüberdrucks platzt.

Es empfiehlt sich indessen, bei Verwirklichung der Erfindung das Substrat Humintorf zu vereinheitlichen. Das geschieht gemäß der Ausführungsform nach Anspruch 2 durch Homogenisierung des Ausgangssubstrates. Hierdurch wird erreicht, daß im Ausgangssubstrat eingeschlossene Gase sich nicht in Form von Blasen sammeln können und daß über die gesamte Ausdehnung des Substrates eine gleichmäßige Wärme- bzw. Kälteabgabe stattfindet.

Der in der Trägersubstanz erfindungsgemäß das Substrat bildende Humintorf wird im wesentlichen aus den unteren, d. h. ältesten und daher am weitestens zersetzten Schichten des Torflagers gewonnen. Dieses organische Material läßt sich gemäß Anspruch 3 mit seiner natürlichen Feuchtigkeit nach Homogenisierung mit dem Kälteträger mischen. Als Kälteträger hat sich 1,2 Propylenglykolalkohol in einer Menge von 35 Gew.% in der Masse des Humintorfs als optimal herausgestellt.

Vorzugsweise weist der Beutel, in dem die mit Kälte oder Wärme aufladbare Trägersubstanz abgefüllt wird, eine Auflageseite und eine aufgeladene Wärme bzw. Kälte in die Trägersubstanz abgebende Außenseite auf. Der Beutel der neuen Kompresse hat erfindungsgemäß nicht nur die Aufgabe, die mit Kälte oder Wärme aufladbare Trägersubstanz flüssigkeitsdicht einzuschließen, sondern soll zugleich einen Thermoeffekt ergeben, der die aufgeladene Kälte oder Wärme auf die Auflageseite der Kompresse konzentriert. Hierdurch werden die Verluste durch nach außen abgestrahlte und therapeutisch nicht nutzbare Wärme oder Kälte praktisch ausgeschlossen, da sich der Beutel wie eine Thermosflasche seiner Umgebung gegenüber verhält.

Vorzugsweise wird die Auflageseite des Beutels gemäß Anspruch 6 transparent ausgeführt, um Wärmeabsorbtion durch das Beutelmaterial zu reduzieren, wobei ferner die dieser Auflageseite gegenüberliegende Beutelseite nach innen reflektierend ausgebildet wird.

Solche Ausführungsformen der erfindungsgemäßen therapeutischen Kalt-Warmkompresse lassen sich gemäß Anspruch 7 auf einfache Weise in die Praxis umsetzen. Der hierbei als Kunststoffolienkissen ausgefüllte Beutel wird aus Folienausschnitten zusammengesetzt, die durch Randverschweißung den gewünschten dampf- und flüssigkeitsdichten Innenraum durch ihre Randverschweißung miteinander bilden. Die Konzentration der von der Trägersubstanz abgegebenen Wärme oder Kälte auf die Auflageseite der Kompresse wird durch die metallische Oberfläche der Metallverbundfolie bewirkt, welche den Folienausschnitt bildet, aus dem die der Auflageseite der Kompresse gegenüberliegende Beutelseite besteht.

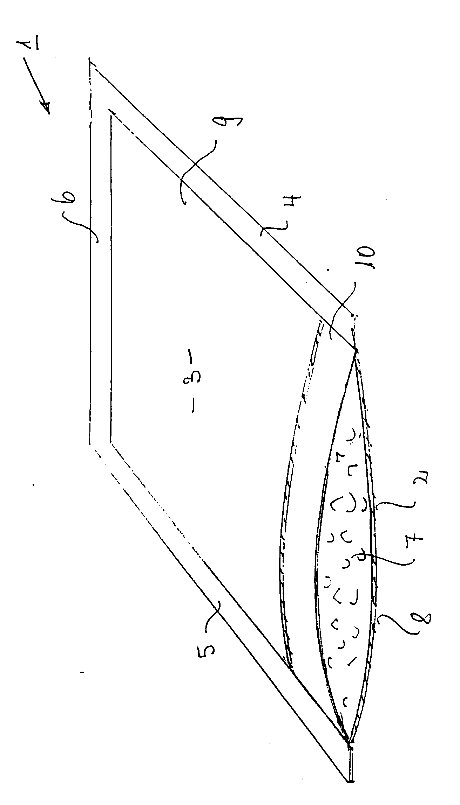

Eine Ausführungsform der Erfindung ist in der Zeichnung dargestellt, die in abgebrochener Darstellung schematisch die neue Kalt-Warmkompresse perspektivisch wiedergibt.

Die in der Zeichnung dargestellte therapeutische Kalt-Warmkompresse 1 besteht gemäß dem Ausführungsbeispiel aus zwei im wesentlichen kongruenten randverschweißten Folienabschnitten 2, 3. Der Grundriß ist rechteckig, so daß sich zwei parallele längere Schweißkanten 4, 5 ergeben, die in zwei kürzere ihrerseits parallele Schweißkanten übergehen, von denen eine bei 6 in der Zeichnung sichtbar ist. Der Innenraum der sich aus der allseitigen Randverschweißung ergibt, ist mit einer mit Kälte oder Wärme aufladbaren Trägersubstanz 7 luftblasenfrei gefüllt. Diese Trägersubstanz besteht gemäß dem dargestellt Ausführungsbeispiel aus homogenisiertem Humintorf, dem ein Anteil von ca. 35 % Glykol zugesetzt ist.

Da das Substrat flüssig ist, ergibt sich ein flexibles Kissen, in dem der Folienausschnitt 2, welcher aus transparierter Kunststoffolie besteht, mit seiner Außenseite 8 die Auflageseite bildet. Der gegenüberliegende Folienausschnitt 3 besteht aus einer Verbundfolie nämlich PAPE-(Nylon)folie 9 mit einer Metallisierungsschicht 10, die aus Aluminium besteht.

Die Kompresse wird vom Hersteller fertig angeliefert und vom Anwender in einem Kühlschrank mit Kälte oder heißem Wasser während ca. 8 Minuten mit Wärme beladen. Die so vorbereitete Kompresse wird vor dem Auflegen auf die Haut in eine Kompressenhülle, beispielsweise ein dünnes Tuch oder ähnliches eingelegt und sodann mit ihrer Auflageseite 8 auf die Haut der zu therapierenden Körperpartie aufgelegt. Die Wärme oder Kälte wird von der Metallisierung 10 in die Trägersubstanz reflektiert und aus dieser durch die transparente Folie 2 auf die Körperpartie abgegeben. Ebenso verhält es sich bei einer Kalttherapie, für die die Kompresse vorher mit Kälte beladen worden ist.

## Patentansprüche

1. Therapeutische Kalt-Warmkompresse, bei der ein flüssigkeitsdichter Beutel (1) mit einer mit Kälte oder Wärme aufladbaren Trägersubstanz (7) gefüllt ist, welche ein flüssiges Substrat aufweist und welche in dem Beutel nach außen dampfdicht abgeschlossen untergebracht ist dadurch gekennzeichnet, daß in der Trägersubstanz (7) das Substrat Humintorf ist, das Substrat mit einem Kältemittel gemischt ist und in dem Beutel (1) luftblasenfrei untergebracht ist.

2. Therapeutische Kalt-Warmkompresse nach Anspruch 1, dadurch gekennzeichnet, daß das Ausgangssubstrat des Humintorfes homogenisiert ist.

3. Therapeutische Kalt-Warmkompresse nach einem der AnSprüche 1 oder 2, dadurch gekennzeichnet, daß das Ausgangssubstrat des Humintorfs mit einem natürlichen Feuchtigkeitsgehalt nach seiner Homogenisierung mit dem Kälteträger gemischt ist.

4. Therapeutische Kalt-Warmkompresse nach einem oder mehreren der Ansprüche 1 bis 3, dadurch gekennzeichnet, daß dem Humintorf 1.2 Propylenglykolalkohol mit einem Anteil von 35 Gew.% beigemischt ist.

5. Therapeutische Kalt-Warmkompresse nach einem oder mehreren der Ansprüche 1 bis 4, dadurch gekennzeichnet, daß der Beutel (1) eine Auflageseite (2) und eine aufgeladene Wärme bzw. Kälte in die Trägersubstanz (7) abgebende Außenseite (9) aufweist.

6. Therapeutische Kalt-Warmkompresse nach Anspruch 5, dadurch gekennzeichnet, daß die Auflageseite (2) des Beutels (1) transparent und die ihr gegenüberliegende Beutelseite nach innen reflektierend ausgebildet ist.

7. Therapeutische Kalt-Warmkompresse nach einem oder mehreren der Ansprüche 5 und 6, dadurch gekennzeichnet, daß der als Kunststoffolienkissen ausgefüllte Beutel (1) im wesentlichen aus zwei kongruenten randverschweißten Folienausschnitten (2, 3) besteht, von denen eine aus einer Metallverbundfolie ausgeschnitten und mit der blanken Metallseite auf den Transparentfolienausschnitt aufgebracht ist.

## Claims

1. Therapeutic hot and cold compress in which a liquid-tight bag (1) is filled with a carrier substance (7) which may be charged with heat or cold and which has a liquid substrate and which is accommodated in the bag sealed from the exterior in a vapour-tight manner, characterized in that the substrate in the carrier substance (7) is humic peat, the substrate is mixed with a refrigerant and is accommodated in the bag (1) without air bubbles.

2. Therapeutic hot and cold compress as claimed in Claim 1, characterised in that the starting substrate of the humic peat is homogenised.

3. Therapeutic hot and cold compress as claimed in one of Claims 1 or 2, characterised in that the starting substrate of the humic peat with a natural moisture content is mixed with the refrigerant after its homogenisation.

4. Therapeutic hot and cold compress as claimed in one or more of Claims 1 to 3, characterised in that 1.2 propylene glycol alcohol is mixed into the humic peat in a proportion of 35 % by wt.

5. Therapeutic hot and cold compress as claimed in one or more of Claims 1 to 4, characterised in that the bag (1) has a support side (2) and an outer side (9) which releases charged heat or cold into the carrier substance (7).

6. Therapeutic hot and cold compress as claimed in Claim 5, characterised in that the support side (2) of the bag (1) is transparent and the side of the bag opposite to it is internally reflective.

7. Therapeutic hot and cold compress as claimed in one or more of Claims 5 and 6, characterised in that the bag (1), which is filled to form a plastic film cushion, substantially comprises two congruent edge welded film blanks (2, 3), one of which is cut out of a composite metallic film and is applied with the exposed metal side to the transparent film blank.

## Revendications

1. Compresse thérapeutique froide-chaude, dans le cas de laquelle un sachet (1), étanche aux liquides, est rempli avec une substance support (7) pouvant emmagasiner du froid ou de la chaleur, Présentant un substrat liquide, et introduite dans le sachet (1) en étant isolée vers l'extérieur de façon à être étanche à la vapeur, caractérisée en ce que, dans la substance support (7), il y a de la tourbe humique, en ce que le substrat est mélangé à un fluide réfrigérant et est placé dans le sachet (1) sans bulles d'air.

2. Compresse thérapeutique froide-chaude suivant la revendication 1, caractérisée en ce que le substrat initial de la tourbe humique est homogénéisé.

3. Compresse thérapeutique froide-chaude suivant l'une des revendications 1 ou 2, caractérisée en ce que le substrat initial de la tourbe humique, comportant une teneur naturelle en humidité, est mélangé, après son homogénéisation, avec le fluide réfrigérant.

4. Compresse thérapeutique froide-chaude suivant l'une ou plusieurs des revendications 1 à 3, caractérisée en ce qu'à la tourbe humique, est mélangé de l'alcool propylène-glycol 1.2 dans une Proportion de 35 % en poids.

5. Compresse thérapeutique froide-chaude suivant l'une ou plusieurs des revendications 1 à 4, caractérisée en ce que le sachet (1) présente une face d'application (2) et une face externe (9) transmettant dans la substance support (7) la chaleur ou le froid emmagasinés.

6. Compresse thérapeutique froide-chaude suivant la revendication 5, caractérisée en ce que la face d'application (2) du sachet (1) est transparente et en ce que la face du sachet qui lui est opposée est réalisée refléchissante vers l'intérieur.

7. Compresse thérapeutique froide-chaude suivant l'une ou plusieurs des revendications 5 et 6, caractérisée en ce que le sachet (1) rempli en formant un coussin de feuilles de matière plastique est essentiellement constitué de deux découpes de feuille (2, 3) congruentes soudées en bordure, dont l'une est découpée dans une feuille composite métallique et est rapportée, avec sa face métallique brillante, sur la découpe de feuille transparente.
